# EUROPEAN PATENT APPLICATION

(11) **EP 4 570 251 A1**
(43) Date of publication of application: **18.06.2025**
(21) Application number: 23840032.9
(22) Date of filing: 17.07.2023
(51) Int. Cl.: A61K 31/5575, A61P 13/12, A23L 33/10

(54) **COMPOSITION FOR TREATING OR PREVENTING KIDNEY DISEASES**

(30) Priority: 15.07.2022 KR 20220087838
(71) Applicant: Organoidsciences Ltd., Seongnam-si, Gyeonggi-do 13493 (KR)
(72) Inventor: YOO, Jongman, Seongnam-si Gyeonggi-do 13540 (KR); CHOI, Woo Hee, Yongin-si Gyeonggi-do 16807 (KR); JOO, Lina, Seoul 04974 (KR); PARK, Jun-Hyeok, Seongnam-si Gyeonggi-do 13504 (KR); LEE, Jaeseon, Seoul 02025 (KR); SONG, Jihyeon, Seongnam-si Gyeonggi-do 13584 (KR)
(74) Representative: Rückerl, Florian
(86) International application number: PCT/KR2023/010229
(87) International publication number: WO 2024/014941

(57) **Abstract**

The present invention relates to a composition for treating, alleviating or preventing kidney diseases and, more particularly, to a pharmaceutical or food composition for treating, alleviating or preventing kidney diseases, containing, as an active ingredient, PGF_{2α}, a PGF_{2α} agonist and/or a PGF_{2α} analogue. The composition containing PGF_{2α}, a PGF_{2α} agonist and/or PGF_{2α} analogue, according to the present invention, can exhibit the effect of treating or preventing kidney damage caused by antibiotics. Particularly, since the effect can be achieved without influencing the antibacterial effectiveness of antibiotics, the composition can be effectively used as a material for treating, alleviating or preventing kidney diseases.

## Description

### [Technical Field]

The present invention relates to a composition for treating, alleviating, or preventing kidney diseases, and more particularly, to a pharmaceutical or food composition for treating, alleviating, or preventing kidney diseases, comprising PGF_{2α}, a PGF_{2α} agonist, and/or a PGF_{2α} analogue as an active ingredient.

### [Background Art]

The kidney is an important organ responsible for excretion. As various substances are excreted through the kidney, certain pharmaceutical substances may instead cause toxicity to the kidney, thereby inducing kidney diseases. A representative example is nephrotoxicity induced by colistin.

Colistin is an antibiotic against Gram-negative bacteria, but it was not used for a period of time due to its side effect of nephrotoxicity. Currently, it is being used as a last-resort treatment for multidrug-resistant Gram-negative bacteria, particularly *Pseudomonas aeruginosa.* The nephrotoxicity caused by colistin occurs due to increased permeability of the tubular epithelial cell membrane, which is known to induce the influx of cations, anions, and water, thereby causing swelling and lysis of kidney cells. In addition, inflammation and oxidation of cells caused by colistin are also involved in the induction of nephrotoxicity.

In the related art, many studies have been conducted to develop drugs for treating or preventing nephrotoxicity caused by colistin. For example, animal studies have been conducted to evaluate the kidney-protective effects by utilizing the antioxidative properties of substances such as N-acetylcysteine and ascorbic acid. However, no drug has yet been developed for clinical application. Currently, no countermeasure exists other than continuously monitoring renal function during colistin administration and promptly discontinuing colistin upon observation of renal function deterioration.

### [Technical Problem]

The inventors conducted studies on drugs capable of preventing or treating kidney diseases, particularly kidney diseases induced by antibiotics. As a result, they confirmed that PGF_{2α}, a PGF_{2α} agonist, and/or a PGF_{2α} analogue exhibit effects of treating or preventing nephrotoxic symptoms induced by antibiotics, without affecting the antibacterial activity of the antibiotics. Accordingly, the present invention was completed by experimentally demonstrating that PGF_{2α}, a PGF_{2α} agonist, and/or a PGF_{2α} analogue can be usefully applied to the treatment of nephrotoxicity or kidney diseases induced by antibiotics.

### [Technical Solution]

Each description and embodiment disclosed in the present invention may be applied to other descriptions and embodiments, respectively. That is, all combinations of various elements disclosed in the present invention fall within the scope of the present invention.

Furthermore, the scope of the present invention should not be construed as being limited by the specific descriptions provided below.

In addition, terms not specifically defined in the present specification shall be understood to have meanings commonly used in the technical field to which the present invention pertains. Also, unless otherwise defined in context, the singular includes the plural, and the plural includes the singular.

One aspect of the present invention provides a pharmaceutical composition for treating or preventing kidney diseases, comprising, as an active ingredient, at least one selected from the group consisting of PGF_{2α}, a PGF_{2α} agonist and a PGF_{2α} analogue.

As used herein, the term "PGF_{2α}" refers to prostaglandin F_{2α}, which is also known as dinoprost. PGF_{2α} is a naturally occurring prostaglandin synthesized in the body.

As used herein, the term "PGF_{2α} receptor" refers to a receptor protein to which PGF_{2α} binds, and is also referred to as the prostaglandin F2-alpha receptor (PGF2Rα), PGF2-alpha receptor, prostaglandin F receptor, PGF receptor, or FP receptor. The PGF_{2α} receptor is encoded by the PTGFR gene, and information on the protein sequence thereof is available from publicly known databases (e.g., NCBI Reference Sequence: NP_000950, NP_001034674).

As used herein, the term "PGF_{2α} agonist" refers to a substance that binds to a PGF_{2α} receptor and activates its action.

As used herein, the term "PGF_{2α} analogue" refers to a compound that is chemically or structurally similar to PGF_{2α}. A PGF_{2α} analogue is considered to bind to the PGF_{2α} receptor (Anne J Lee, Clin Ophthalmol. 2010, 4: 741-764).

Specifically, the PGF_{2α} agonist or PGF_{2α} analogue may be at least one selected from the group consisting of latanoprost, bimatoprost, unoprostone, carboprost, tafluprost and travoprost.

It is well known in the art that latanoprost, bimatoprost, unoprostone, carboprost, tafluprost, and travoprost are PGF_{2α} analogues (C B Camras et al., Ophthalmology. 1996 Nov; 103(11):1916-24; Jae Young Heo et al., Exp Eye Res. 2020 May; 194:108019; Marc Bygdeman, Best Pract Res Clin Obstet Gynaecol. 2003 Oct; 17(5):707-16; Takashi Ota et al., Invest Ophthalmol Vis Sci. 2005 Jun; 46(6):2006-11).

In the present invention, the kidney disease may be caused by administration of an antibiotic. As a specific example, the antibiotic may be at least one selected from the group consisting of colistin, erythromycin, ampicillin, polymyxin B, tetracycline, kanamycin, neomycin, tylosin, levofloxacin, methicillin, imipenem, amikacin and derivatives thereof, but is not limited thereto.

In addition, the antibiotic may be one that targets Gram-negative bacteria. The antibiotics targeting Gram-negative bacteria may be at least one selected from the group consisting of colistin, carbapenem, meropenem, imipenem, ertapenem and derivatives thereof, but are not limited thereto.

In the present invention, the kidney disease may be induced by nephrotoxicity. As used herein, the term "nephrotoxicity" refers to a property of causing damage to the kidney by a specific drug or toxic substance.

In the present invention, the kidney disease may be at least one selected from the group consisting of nephritis, pyelitis, nephrotic syndrome, kidney cancer, acute pyelonephritis, chronic pyelonephritis, renal tuberculosis, urinary tract infection, urolithiasis, ureterolithiasis, acute renal failure, chronic renal failure, diabetic nephropathy, chronic glomerulonephritis, rapidly progressive glomerulonephritis, nephrotic syndrome, focal segmental glomerulosclerosis, membranous nephropathy and membranoproliferative glomerulonephritis, but is not limited thereto.

In the present invention, the composition may inhibit or prevent the generation of reactive oxygen species or apoptosis, and may improve or alleviate at least one symptom selected from the group consisting of body weight loss in a subject, change in kidney color, increased blood urea nitrogen (BUN) level, increased blood creatinine (Cre) level, and increased expression of kidney injury marker-1 (KIM-1).

As used herein, the term "treatment" refers to any act of treating, improving, alleviating, or curing nephrotoxicity or a kidney disease through administration of the composition according to the present invention. In addition, the term "prevention" refers to any act of suppressing or delaying the occurrence of nephrotoxicity or a kidney disease, or suppressing or delaying the occurrence or progression of symptoms caused by nephrotoxicity or a kidney disease through administration of the pharmaceutical composition according to the present invention.

The pharmaceutical composition of the present invention may further comprise a suitable carrier, excipient, or diluent that is conventionally used in the manufacture of pharmaceutical compositions. Examples of carriers, excipients, and diluents that may be contained in the composition may be, but are not limited to, lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, or mineral oil.

The pharmaceutical composition according to the present invention may be formulated for administration simultaneously, separately, or sequentially with other substances or components. For such administration, PGF_{2α}, a PGF_{2α} agonist, and/or a PGF_{2α} analogue comprised in the composition of the present invention and the other substance or component may be formulated in separate containers or in a single container.

As used herein, the term "administration" refers to the physical introduction of the composition into a subject using any of various methods and delivery systems known to those skilled in the art.

The pharmaceutical composition of the present invention may be formulated into an oral preparation or a non-oral preparation, depending on the route of administration. Further, the pharmaceutical composition of the present invention may be formulated into a suitable dosage form for topical administration using techniques well known to those skilled in the art, and the topical dosage form may include external preparations, effervescent tablets, suppositories, and the like. In one embodiment, the pharmaceutical composition of the present invention may be formulated as an external preparation by mixing the PGF_{2α}, PGF_{2α} agonist, and/or PGF_{2α} analogue with a base that is well known and commonly used in the relevant technical field. The external preparation may include an emulsion, gel, ointment, cream, patch, liniment, powder, aerosol, spray, lotion, serum, paste, foam, drop, suspension, and/or tincture.

In the case of oral preparations, the composition may be formulated using methods known in the art into powders, granules, tablets, pills, sugar-coated tablets, capsules, liquids, gels, syrups, suspensions, and the like. For example, tablets or sugar-coated tablets may be obtained by mixing the active ingredient with a solid excipient, pulverizing the mixture, adding a suitable auxiliary agent, and processing the result into a granule mixture. Suitable excipients may include sugars such as lactose, dextrose, sucrose, sorbitol, mannitol, xylitol, erythritol, and maltitol; starches such as corn starch, wheat starch, rice starch, and potato starch; celluloses such as cellulose, methyl cellulose, sodium carboxymethylcellulose, and hydroxypropylmethylcellulose; and fillers such as gelatin and polyvinylpyrrolidone. In some cases, disintegrants such as cross-linked polyvinylpyrrolidone, agar, alginic acid, or sodium alginate may also be added. Furthermore, the composition may additionally comprise anti-aggregation agent, lubricants, wetting agents, flavoring agents, emulsifiers, or preservatives. Liquid preparations for oral administration may include suspensions, oral solutions, emulsions, and syrups, which may contain various excipients, such as wetting agents, sweeteners, flavoring agents, and preservatives, in addition to simple diluents such as water and liquid paraffin. Preparations for non-oral administration may include sterile aqueous solutions, non-aqueous solvents, suspensions, emulsions, lyophilized preparations, and suppositories.

Another aspect of the present invention provides a method for treating or preventing a kidney disease, comprising administering the pharmaceutical composition to a subject.

In the method for treating or preventing a kidney disease according to the present invention, each term has the same meaning as described above unless otherwise specified.

The subject refers to a human or animal, and as used herein, the term "animal" refers to any non-human animal. The non-human animal may include, for example, vertebrates such as primates, dogs, cattle, horses, pigs, chickens, ducks, geese, rodents such as mice, rats, and guinea pigs. As used herein, the term "subject" may be used interchangeably with "individual" or "patient".

The pharmaceutical composition may be administered in an effective amount to a subject in need thereof to improve nephrotoxicity or a kidney disease, or to treat or prevent symptoms caused by nephrotoxicity or a kidney disease.

In addition, the pharmaceutical composition may be administered in an effective amount to a subject in need thereof to improve nephrotoxicity or a kidney disease induced by an antibiotic, or to treat or prevent symptoms caused by the nephrotoxicity or kidney disease induced by an antibiotic.

The effective amount may be a "therapeutically effective amount" or a "prophylactically effective amount". As used herein, the term "therapeutically effective amount" refers to any amount which, when the drug or therapeutic agent is used alone or in combination with other therapeutic agents, results in a reduction in symptom severity, an increase in frequency or duration of symptom-free periods, or prevention of impairment or disability caused by symptoms. As used herein, the term "prophylactically effective amount" refers to any amount that can prevent, suppress, or delay the risk of occurrence or recurrence of the condition. The level of the effective amount may be determined by a person skilled in the art, such as a medical doctor, taking into consideration factors such as severity, age, sex, potency, sensitivity to the drug, time and route of administration, excretion rate, duration of treatment, concurrent medications, and other factors well known in the medical field.

The route of administration for the pharmaceutical composition of the present invention includes, for example, oral administration or non-oral administration such as intravenous, intramuscular, subcutaneous, intraperitoneal, spinal, or other routes of injection or infusion, but is not limited thereto.

The number of administrations of the composition of the present invention may be, for example, once, multiple times, and/or over one or more extended periods. In addition, the frequency of administration may vary depending on the route of administration, severity of the disease, sex, body weight, age, dosage, and other factors.

The pharmaceutical composition of the present invention may be administered to a subject in a pharmaceutically effective amount. As used herein, the term "pharmaceutically effective amount" refers to an amount sufficient to treat a disease at a benefit/risk ratio applicable to medical treatment, and the effective dosage level may be determined depending on factors such as the type and severity of the subject, age, sex, activity of the drug, sensitivity to the drug, time and route of administration, excretion rate, duration of treatment, concurrent medications, and other factors well known in the medical field.

The pharmaceutical composition according to the present invention may be administered in combination with other substances or components. In such a case, the composition of the present invention and the other substances or components may be administered sequentially, simultaneously, or separately, and may be administered once or multiple times. The other substances or components may be contained without limitation as long as they do not inhibit or offset the efficacy of PGF_{2α}, the PGF_{2α} agonist, and/or the PGF_{2α} analogue. In consideration of all the above factors, it is important to administer the minimal effective amount to achieve the maximum therapeutic effect without side effects, which may be readily determined by those skilled in the art.

In addition, PGF_{2α}, the PGF_{2α} agonist, and/or the PGF_{2α} analogue comprised in the composition of the present invention and other substances or components may have the same or different effective amount, administration time, administration interval, or route of administration. In the method according to the present invention, the PGF_{2α}, the PGF_{2α} agonist, and/or the PGF_{2α} analogue may be administered to a subject simultaneously, sequentially, or separately with other substances or components. The term "simultaneous administration" means administering PGF_{2α}, the PGF_{2α} agonist, and/or the PGF_{2α} analogue and other substances as a single formulation at the same time, or administering them as separate formulations at the same time, in which case the routes of administration of PGF_{2α}, the PGF_{2α} agonist, and/or the PGF_{2α} analogue and the other substances may differ. The term "sequential administration" refers to administering PGF_{2α}, the PGF_{2α} agonist, and/or the PGF_{2α} analogue and other substances in a relatively continuous manner, allowing the minimum possible time lapse between administrations. The term "separate administration" refers to administering PGF_{2α}, the PGF_{2α} agonist, and/or the PGF_{2α} analogue and the other substances at a certain time interval. The administration method of PGF_{2α}, the PGF_{2α} agonist, and/or the PGF_{2α} analogue and the other substances may be appropriately selected by a physician or expert in the field, taking into account the therapeutic efficacy and side effects in the subject.

Another aspect of the present invention provides a food composition for alleviating or preventing kidney diseases, comprising, as an active ingredient, at least one selected from the group consisting of PGF_{2α}, a PGF_{2α} agonist, and a PGF_{2α} analogue.

In the food composition according to the present invention, each term has the same meaning as described above unless otherwise specified.

As used herein, the term "alleviating" refers to any act by which nephrotoxicity or a kidney disease is prevented from worsening and maintained at a certain level, or symptoms caused by nephrotoxicity or a kidney disease are at least reduced, alleviated, or rendered favorable by administration of the composition according to the present invention.

The food composition may be ingested in various forms and may be provided as a liquid formulation such as a tea or beverage, or as any one formulation selected from the group consisting of pills, tablets, granules, powders and capsules, but is not limited thereto.

The type of food is not particularly limited. Examples of foods into which the food composition according to the present invention may be commercialized or formulated include, but are not limited to, yogurt, dairy products, meat, sausage, bread, chocolate, candies, snacks, confectioneries, pizza, noodles, chewing gum, ice cream products, dairy products, soups, beverages, teas, drinks, alcoholic beverages, and vitamin complexes, as well as all foods in the ordinary sense.

The food composition of the present invention may additionally comprise various sweeteners or natural carbohydrates like conventional foods, and there is no limitation on the type thereof as long as the additional component does not inhibit or offset the effect of the active ingredient according to the present invention. The natural carbohydrate may be, for example, a monosaccharide such as glucose or fructose; a disaccharide such as maltose or sucrose, a polysaccharide such as dextrin or cyclodextrin; or a sugar alcohol such as xylitol, sorbitol, or erythritol. The sweetener may be, for example, a natural sweetener such as thaumatin or stevia extract, or a synthetic sweetener such as saccharin or aspartame. In addition, the food composition may further comprise one or more carriers, excipients, or diluents.

In the case where the food composition is a beverage composition, there is no particular limitation on the liquid component other than containing PGF_{2α}, a PGF_{2α} agonist, and/or a PGF_{2α} analogue as an active ingredient, and the beverage may further comprise various flavoring agents or natural carbohydrates like conventional beverages.

The food may be a health functional food. As used herein, the term "health functional food" refers to a food prepared and processed in the form of tablets, capsules, powders, granules, liquids, pills, etc., using raw materials or components having functionality beneficial to the human body. The term "functionality" means an effect useful for health purposes such as regulating nutrients or exerting physiological actions on the structure or function of the human body.

In the case where the food composition of the present invention is an animal feed composition, there is no particular limitation other than containing PGF_{2α}, a PGF_{2α} agonist, and/or a PGF_{2α} analogue as an active ingredient. The animal feed may be provided in the form of food, snacks, or dietary supplements for animals in order to improve nephrotoxicity in animals, or to improve or prevent kidney diseases resulting from nephrotoxicity.

The composition comprising PGF_{2α}, a PGF_{2α} agonist, and/or a PGF_{2α} analogue according to the present invention can exhibit therapeutic or preventive effects against kidney damage induced by antibiotics. In particular, such effects can be achieved without affecting the antibacterial efficacy of the antibiotics, and thus the composition can be usefully employed as a substance for treating, alleviating or preventing kidney diseases.

### [Brief Description of Figures]

FIG. 1 is a graph showing the cell viability of a human kidney-2 (HK-2) cell line treated with PGF_{2α}.
FIG. 2 is a graph showing the cell viability of a human kidney-2 cell line pretreated with PGF_{2α} and then treated with colistin. * indicates P value < 0.05.
FIG. 3 is a graph showing the cell viability of a human kidney-2 cell line treated simultaneously with PGF_{2α} and colistin. * indicates P value < 0.05.
FIG. 4 is an image showing apoptosis in a human kidney-2 cell line treated with PGF_{2α} and/or colistin.
FIG. 5 is a graph showing apoptosis in a human kidney-2 cell line treated with PGF_{2α} and/or colistin. ** indicates P value < 0.01.
FIG. 6 is a graph showing the generation of reactive oxygen species (ROS) in a human kidney-2 cell line treated with PGF_{2α} and/or colistin. * indicates P value < 0.05.
FIG. 7 is a graph showing the cell viability of a human kidney-2 cell line treated with bimatoprost.
FIG. 8 is a graph showing the cytotoxicity of a human kidney-2 cell line pretreated with bimatoprost and then treated with colistin. *** indicates P value < 0.001.
FIG. 9 is a graph showing the cytotoxicity of a human kidney-2 cell line treated simultaneously with bimatoprost and colistin. * indicates P value < 0.05; and *** indicates P value < 0.001.
FIG. 10 is an image showing apoptosis in a human kidney-2 cell line treated with bimatoprost and/or colistin.
FIG. 11 is a graph showing apoptosis in a human kidney-2 cell line treated with bimatoprost and/or colistin.
FIG. 12 is a graph showing the generation of reactive oxygen species (ROS) in a human kidney-2 cell line treated with bimatoprost and/or colistin. * indicates P value < 0.05.
FIG. 13 is an image showing the antibacterial activity of bimatoprost and/or colistin against *Escherichia coli.*
FIG. 14 is a graph showing the antibacterial activity of bimatoprost and/or colistin against *Escherichia coli.*
FIG. 15 is a schematic diagram illustrating the establishment of a colistin-induced nephrotoxicity model using 6-week-old C57BL/6N mice.
FIG. 16 is an image showing the kidneys of mice treated with bimatoprost and/or colistin.
FIG. 17 is a graph showing the body weight of mice treated with bimatoprost and/or colistin.
FIG. 18 is a graph showing the blood urea nitrogen (BUN) level in mice treated with bimatoprost and/or colistin.
FIG. 19 is a graph showing the blood creatinine level in mice treated with bimatoprost and/or colistin.
FIG. 20 is an image showing the expression of kidney injury marker-1 (KIM-1) in mice treated with bimatoprost and/or colistin. LTL (Lotus tetragonolobus lectin) is a proximal tubule marker; KIM-1 (Kidney injury marker-1) is a kidney injury marker; and DAPI (4',6-diamidino-2-phenylindole) indicates total cell number.
FIG. 21 is a graph showing the expression level of KIM-1 in mice treated with bimatoprost and/or colistin.

### [Examples]

Hereinafter, specific examples will be described to aid the understanding of the present invention. However, the following examples should not be construed as limiting the scope of the present invention, and conventional modifications may be made by those skilled in the art within the scope of the present invention.

### Example 1. Inhibitory Effect of PGF_{2α} on Nephrotoxicity

### Example 1-1. Protective Effect Against Cytotoxicity

To confirm the effect of PGF_{2α} on kidney diseases, the effect of PGF_{2α} on antibiotic-induced cytotoxicity was evaluated. This was examined by analyzing whether PGF_{2α} can inhibit or protect against cytotoxicity in kidney cells induced by antibiotics.

Specifically, the human renal tubule cell line HK-2 was treated with PGF_{2α} at a concentration of 50 µM and with colistin at 400 µg/mL. In the case of pretreatment with PGF_{2α}, colistin was treated 24 hours after treatment with PGF_{2α}, and the cells were cultured for 48 hours, followed by an assay to evaluate cytotoxicity. In the case of co-treatment, PGF_{2α} and colistin were treated simultaneously, and the cells were cultured for 48 hours, followed by the assay.

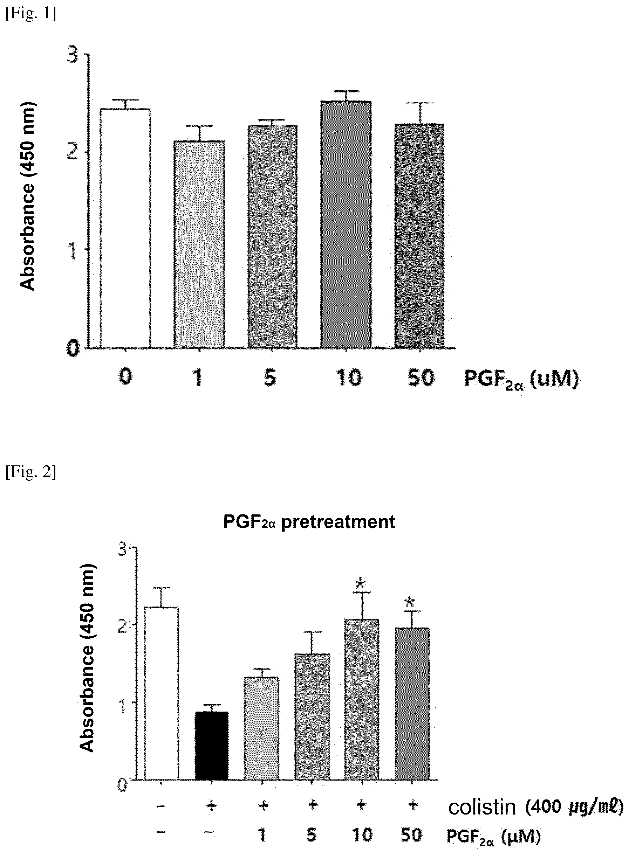

As shown in FIG. 1, PGF_{2α} did not exhibit cytotoxicity toward HK-2 cells. PGF_{2α} did not show cytotoxicity at any of the concentrations tested, and particularly, it was confirmed that no cytotoxicity was observed even at the high concentration of 50 µM.

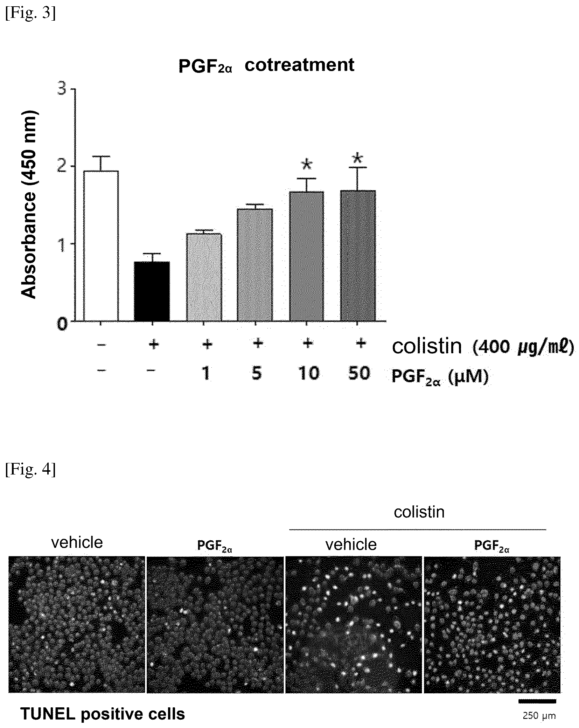

However, as shown in FIGs. 2 and 3, colistin caused cytotoxicity in HK-2 cells, and in cells treated with colistin, only about 50% of the cells survived compared to those not treated with colistin. In contrast, when PGF_{2α} and colistin were co-treated, the cytotoxicity caused by colistin was reduced, and this effect was observed in a PGF_{2α} concentration-dependent manner, regardless of the treatment order of PGF_{2α} and colistin.

These results suggest that PGF_{2α} exhibits an effect of inhibiting or protecting against cytotoxicity in kidney cells, and therefore can be usefully applied for the treatment or prevention of kidney diseases.

### Example 1-2. Inhibitory Effect on Apoptosis

To evaluate the effect of PGF_{2α} on kidney diseases, the influence of PGF_{2α} on apoptosis in kidney cells induced by antibiotic treatment was assessed. Colistin, a type of antibiotic, is known to accumulate in proximal tubular cells and induce apoptosis by increasing the expression of the Fas death receptor or by increasing the generation of reactive oxygen species.

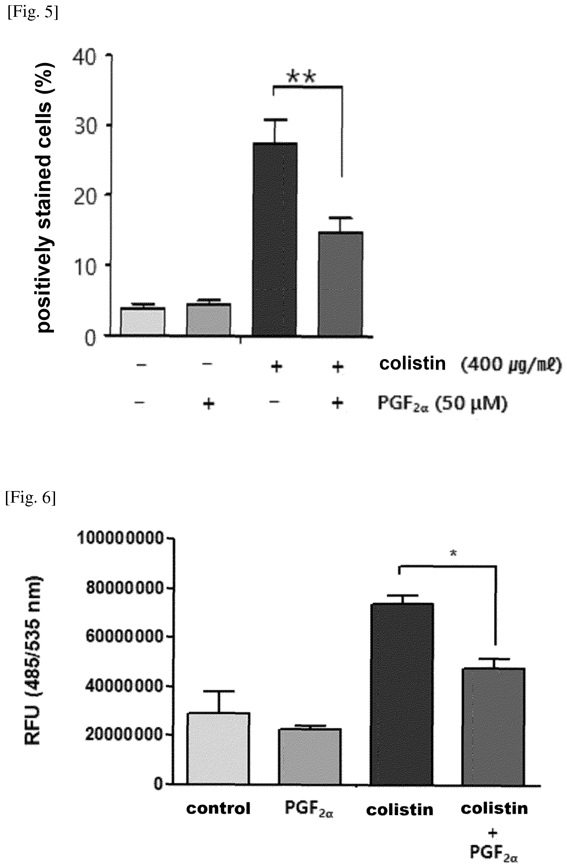

Specifically, the human renal tubule cell line HK-2 was treated with PGF_{2α} at a concentration of 50 µM and with colistin at 400 µg/mL. After 48 hours of incubation, a TUNEL assay was conducted to assess apoptosis. Cells that tested positive in the TUNEL assay were considered apoptotic cells. The result images are shown in FIG. 4, and the corresponding graph is presented in FIG. 5.

As shown in FIGs. 4 and 5, PGF_{2α} treatment alone did not induce apoptosis in HK-2 cells. However, treatment with colistin increased apoptosis by approximately 30% compared to cells not treated with colistin. In contrast, when PGF_{2α} and colistin were co-treated, the colistin-induced increase in apoptosis was reduced by about half.

These results suggest that PGF_{2α} has an inhibitory effect on apoptosis in kidney cells and can therefore be usefully applied for the treatment or prevention of kidney diseases.

### Example 1-3. Inhibitory Effect on Reactive Oxygen Species Generation

To evaluate the effect of PGF_{2α} on kidney diseases, the influence of PGF_{2α} on the generation of reactive oxygen species (ROS) in kidney cells induced by antibiotic treatment was assessed. Colistin, which is a type of antibiotic, promotes the generation of ROS in cells. Accordingly, infiltration of inflammatory cells occurs, and NF-κB, IL-1β and tumor necrosis factor-alpha, which are involved in pro-inflammatory cytokine responses, become activated, leading to inflammation.

Specifically, the human renal tubule cell line HK-2 was treated with PGF_{2α} at a concentration of 50 µM and with colistin at 400 µg/mL. After 48 hours of incubation, ROS generated in the HK-2 cells was analyzed.

As shown in FIG. 6, ROS levels decreased in HK-2 cells treated with PGF_{2α} alone. In contrast, treatment with colistin increased ROS levels by more than fivefold compared to cells not treated with colistin. However, when PGF_{2α} and colistin were co-treated, the colistin-induced increase in ROS was reduced by approximately 1.5-fold.

These results suggest that PGF_{2α} has an inhibitory effect on ROS generation in kidney cells and can therefore be usefully applied for the treatment or prevention of kidney diseases.

### Example 2. Inhibitory Effect of PGF_{2α} Agonists or Analogues on Nephrotoxicity

### Example 2-1. Protective Effect Against Cytotoxicity

Based on the results of Example 1, which confirmed that PGF_{2α} can prevent or treat nephrotoxicity or antibiotic-induced nephrotoxicity, it was further investigated whether compounds known as PGF_{2α} agonists (i.e., FP receptor agonists) and PGF_{2α} analogues exhibit the same effect. Bimatoprost was used as the PGF_{2α} agonist or analogue, and colistin was used as the antibiotic.

Specifically, bimatoprost was treated to human renal tubule cell line HK-2 at concentrations of 0.1 µM, 0.5 µM, 1.0 µM, 5.0 µM, 10 µM, 20 µM, and 50 µM, and the vehicle-treated group was used as a control. In the case of pretreatment with bimatoprost, colistin was treated 24 hours after bimatoprost treatment, followed by incubation for 48 hours and a cytotoxicity assay. In the case of co-treatment, bimatoprost and colistin were treated simultaneously, followed by 48 hours of incubation and the cytotoxicity assay.

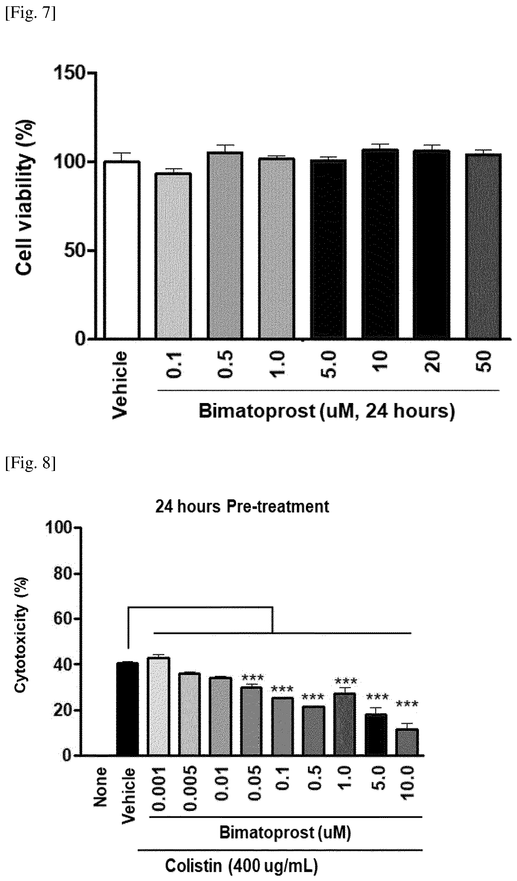

As shown in FIG. 7, bimatoprost did not exhibit cytotoxicity at any of the concentrations tested, and particularly did not exhibit cytotoxicity even at the high concentration of 50 µM.

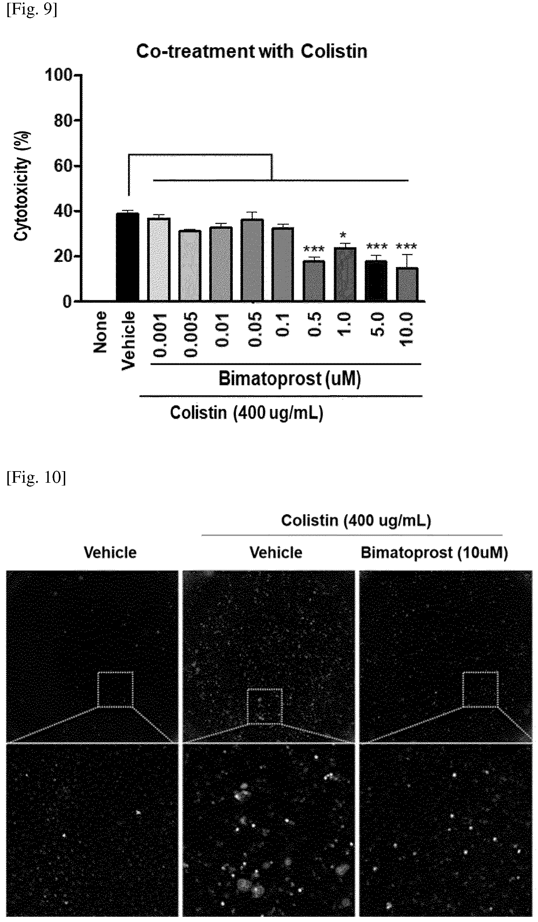

In contrast, as shown in FIGs. 8 and 9, pretreatment with bimatoprost reduced colistin-induced cell death. In particular, when bimatoprost was pretreated at 10 µM, the extent of cell death induced by colistin was reduced by approximately fourfold compared to cells not treated with bimatoprost.

These results suggest that PGF_{2α} agonists or analogues exhibit an inhibitory or protective effect against cytotoxicity in kidney cells and can thus be usefully applied for the treatment or prevention of kidney diseases.

### Example 2-2. Inhibitory Effect on Apoptosis

To evaluate the effect of PGF_{2α} agonists or analogues on kidney diseases, the influence of PGF_{2α} agonists or analogues on apoptosis in kidney cells induced by antibiotic treatment was assessed. Bimatoprost was used as the PGF_{2α} agonist or analogue, and colistin was used as the antibiotic.

Specifically, a vehicle-treated group, a group treated with vehicle and colistin, and a group treated with bimatoprost and colistin were prepared. Apoptosis in HK-2 cells was analyzed using a TUNEL assay.

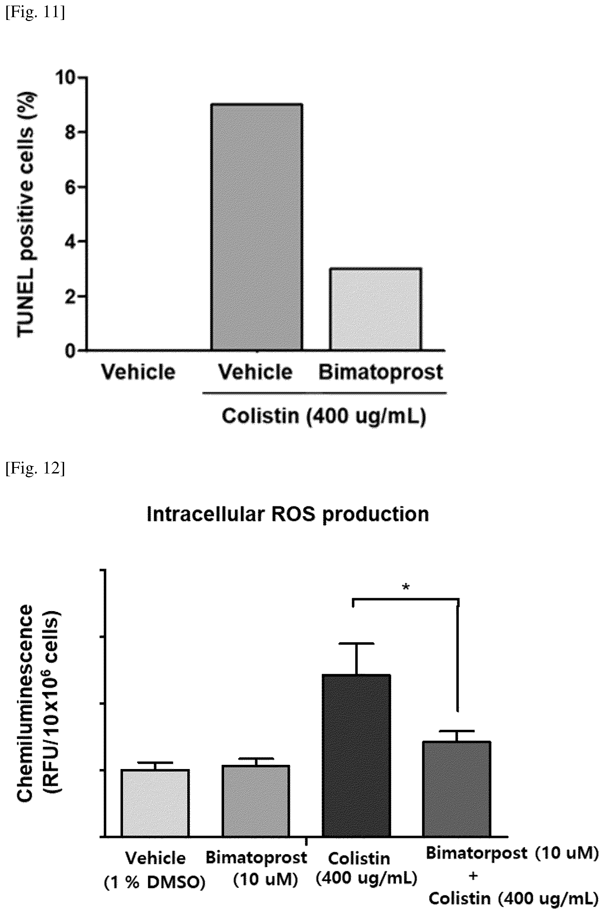

As shown in FIGs. 10 and 11, apoptosis was not observed in the vehicle-treated group, whereas a significant increase in apoptotic cells was observed in the group treated with vehicle and colistin. In contrast, when bimatoprost and colistin were co-treated, the number of apoptotic cells was reduced by approximately threefold compared to the group treated with vehicle and colistin.

These results suggest that PGF_{2α} agonists or analogues exhibit an inhibitory effect on apoptosis in kidney cells and can therefore be usefully applied for the treatment or prevention of kidney diseases.

### Example 2-3. Inhibitory Effect on Reactive Oxygen Species Generation

To evaluate the effect of a PGF_{2α} agonist or analogue on kidney diseases, the influence of PGF_{2α} agonist or analogue on reactive oxygen species generation in kidney cells induced by antibiotic treatment was assessed. Bimatoprost was used as the PGF_{2α} agonist or analogue, and colistin was used as the antibiotic.

Specifically, intracellular reactive oxygen species (ROS) levels were measured by fluorescence spectroscopy after treatment with bimatoprost and/or colistin. Saline containing 1% DMSO was used as a vehicle.

As shown in FIG. 12, ROS levels in the colistin-treated group increased by more than 2.5-fold compared to the vehicle-treated group. In contrast, in the group treated with both bimatoprost and colistin, the colistin-induced increase in ROS was reduced to approximately half, showing a level similar to that of the vehicle-treated group.

These results suggest that PGF_{2α} agonists or analogues exhibit an inhibitory effect on ROS generation in kidney cells, and can thus be usefully applied for the treatment or prevention of kidney diseases.

### Example 2-4. Maintenance of Antibacterial Activity of Antibiotics

It was evaluated whether PGF_{2α} agonists or analogues interfere with the antibacterial activity of antibiotics. Bimatoprost was used as the PGF_{2α} agonist or analogue, and colistin was used as the antibiotic.

Specifically, *Escherichia coli,* a Gram-negative bacterium, was spread onto an LB medium, and paper disks were treated with bimatoprost and/or colistin. Saline containing 1% DMSO was used as a vehicle. After placing the paper disks onto the LB medium, incubation was carried out for 24 hours.

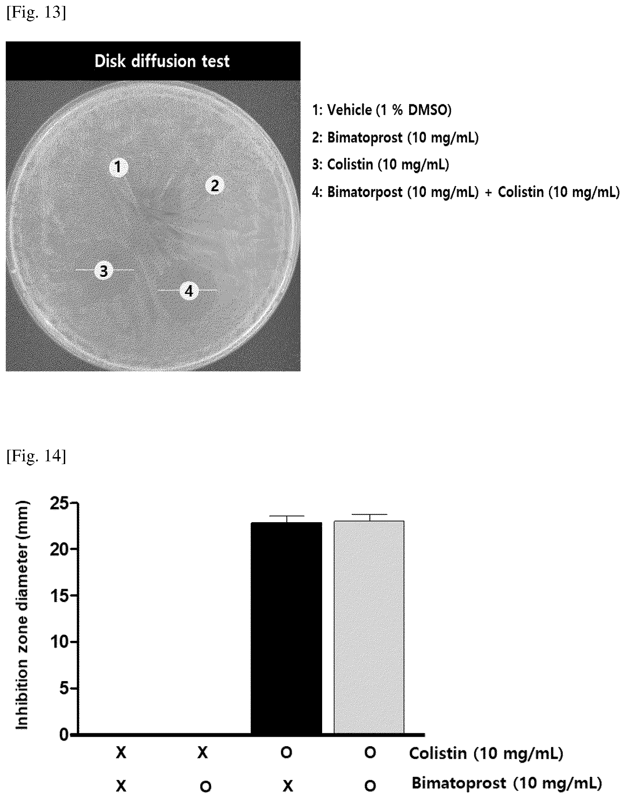

As shown in FIGs. 13 and 14, the growth of *E. coli* was not inhibited in the groups treated with vehicle or bimatoprost alone, whereas the growth of *E. coli* was markedly suppressed in the colistin-treated group. Notably, the inhibitory effect on *E. coli* growth in the group treated with both colistin and bimatoprost was at the same level as that observed in the group treated with colistin alone.

These results suggest that PGF_{2α} agonists or analogues do not interfere with the antibacterial activity of antibiotics, and alleviate only side effects such as antibiotic-induced nephrotoxicity. Therefore, they can be usefully applied for the treatment or prevention of kidney diseases induced by antibiotics.

### Example 2-5. Effect in Animal Model

To evaluate the effect of a PGF_{2α} agonist or analogue on kidney diseases, the *in vivo* effect of PGF_{2α} agonist or analogue on antibiotic-induced nephrotoxicity was investigated. Bimatoprost was used as the PGF_{2α} agonist or analogue, and colistin was used as the antibiotic.

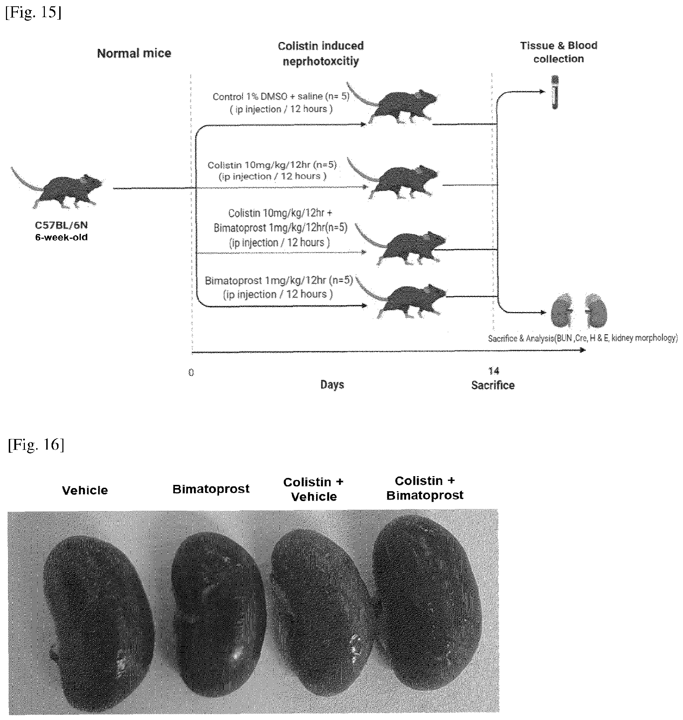

Specifically, a colistin-induced nephrotoxicity model was established using 6-week-old C57BL/6N mice. The control group (vehicle-treated group) was prepared by intraperitoneally injecting mice with vehicle (saline containing 1% DMSO) every 12 hours for 14 days. The colistin-treated group was prepared by intraperitoneally injecting colistin at 10 mg/kg every 12 hours for 14 days. The bimatoprost-treated group was prepared by intraperitoneally injecting bimatoprost at 1 mg/kg every 12 hours for 14 days. The bimatoprost and colistin co-treatment group was prepared by intraperitoneally injecting bimatoprost at 1 mg/kg and colistin at 10 mg/kg every 12 hours for 14 days. An overview of the experimental procedure is shown in FIG. 15.

After 14 days of administration, tissue, blood, and kidneys were collected from the mice, and key indicators of kidney disease, including kidney color, body weight, blood urea nitrogen levels, blood creatinine levels, and expression of kidney injury marker-1 (KIM-1), were analyzed.

### Example 2-5-1. Change in Kidney Color

As shown in FIG. 16, the kidney color in the vehicle-treated group and the bimatoprost-treated group was similar, whereas that in the colistin-treated group became paler, indicating that toxicity had occurred. In contrast, in the group treated with both bimatoprost and colistin, the kidney color recovered to a level similar to that of the vehicle-treated group, and colistin-induced nephrotoxicity was alleviated.

These results suggest that PGF_{2α} agonists or analogues exhibit an effect of alleviating kidney cell damage, and can therefore be usefully applied for the treatment or prevention of kidney diseases.

### Example 2-5-2. Change in Body Weight

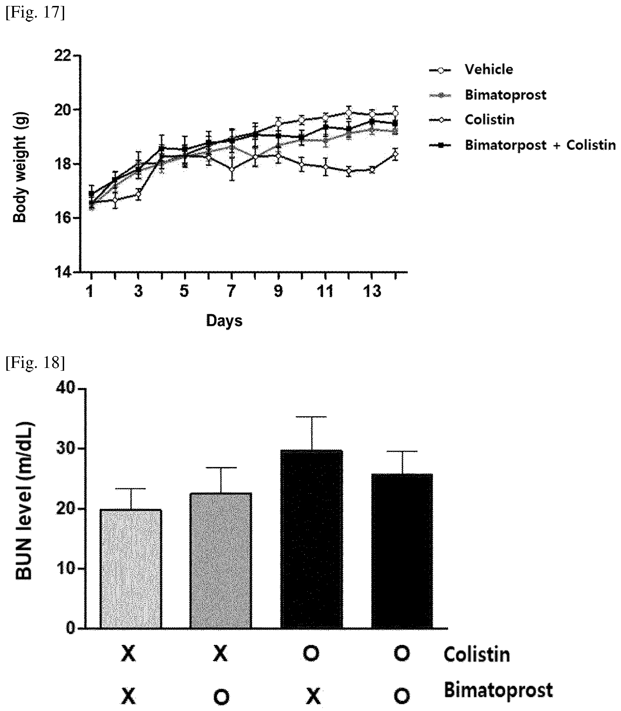

As shown in FIG. 17, body weight changes in the vehicle-treated group and the bimatoprost-treated group were similar, whereas the colistin-treated group showed a reduction in body weight after day 6. In contrast, in the group treated with both bimatoprost and colistin, body weight changes were similar to those of the vehicle-treated group, and weight loss was not observed.

These results suggest that PGF_{2α} agonists or analogues mitigate kidney cell damage and exhibit a weight loss-suppressing effect, and can therefore be usefully applied for the treatment or prevention of kidney diseases.

### Example 2-5-3. Change in Blood Urea Nitrogen

When renal excretory function is impaired, filtration becomes insufficient, leading to an increase in the concentration of urea nitrogen in the blood. Therefore, blood urea nitrogen (BUN) levels are used as one of the important indicators for evaluating the efficacy of therapeutic agents for kidney diseases.

As shown in FIG. 18, the blood urea nitrogen levels in the vehicle-treated group and the bimatoprost-treated group were similar. In contrast, the colistin-treated group showed the blood urea nitrogen levels approximately 1.5 times higher than those in the vehicle-treated group.

However, in the group treated with both bimatoprost and colistin, the blood urea nitrogen levels were lower than those in the colistin-treated group.

These results suggest that PGF_{2α} agonists or analogues alleviate kidney cell damage and exhibit an effect of restoring the excretory and filtration functions of the kidney, and can therefore be usefully applied for the treatment or prevention of kidney diseases.

### Example 2-5-4. Change in Blood Creatinine

Similar to blood urea nitrogen, blood creatinine concentration increases due to impaired renal filtration function and is therefore used as one of the important indicator for evaluating the efficacy of therapeutic agents for kidney diseases.

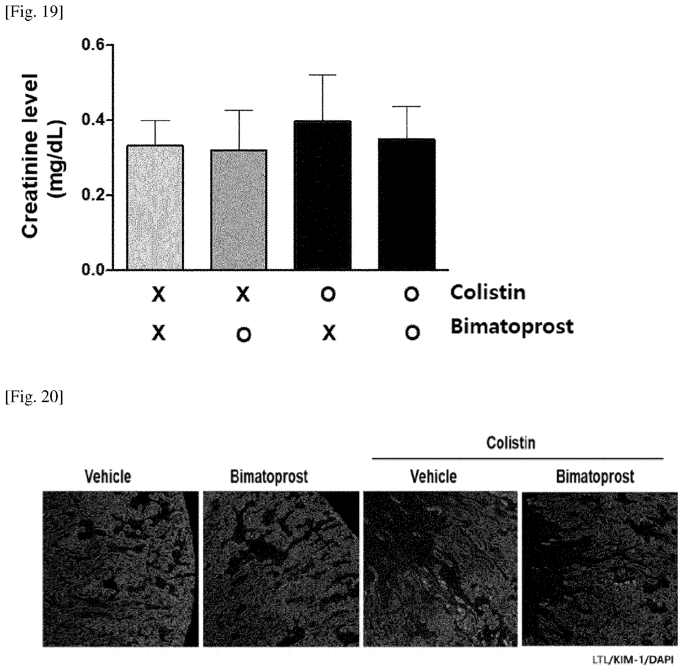

As shown in FIG. 19, the blood creatinine levels in the vehicle-treated group and the bimatoprost-treated group were similar. In contrast, the colistin-treated group exhibited blood creatinine levels approximately 1.5 times higher than those in the vehicle-treated group. However, in the group treated with both bimatoprost and colistin, blood creatinine levels were lower than those in the colistin-treated group.

These results suggest that PGF_{2α} agonists or analogues alleviate kidney cell damage and exhibit an effect of restoring the excretory and filtration functions of the kidney, and can therefore be usefully applied for the treatment or prevention of kidney diseases.

### Example 2-5-5. Expression of Kidney Injury Marker

Kidney injury marker-1 (KIM-1) is not detected in normal kidneys, but is known to show increased expression in dedifferentiated proximal tubules when acute kidney injury occurs due to ischemia or nephrotoxic agents.

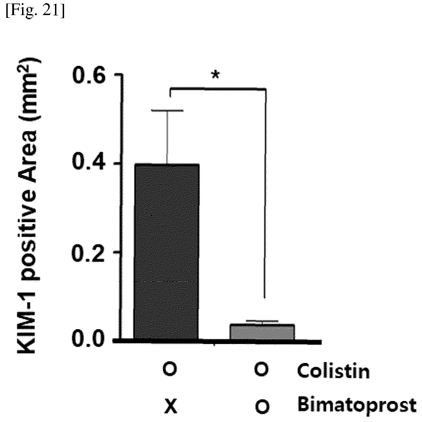

As shown in FIGs. 20 and 21, KIM-1 expression was not observed in the vehicle-treated group or the bimatoprost-treated group. In contrast, KIM-1 expression was clearly observed in the colistin-treated group, and the area was also broad. However, in the group treated with both bimatoprost and colistin, KIM-1 expression was barely observed, and the area was reduced by about eight times compared to the colistin-treated group.

These results suggest that PGF_{2α} agonists or analogues alleviate kidney cell damage and exhibit an effect of restoring kidney tissue, and can therefore be usefully applied for the treatment or prevention of kidney diseases.

## Claims

1. A pharmaceutical composition for treating or preventing kidney diseases, comprising, as an active ingredient, at least one selected from the group consisting of PGF_{2α}, a PGF_{2α} agonist and a PGF_{2α} analogue.

2. The pharmaceutical composition of claim 1, wherein the PGF_{2α} agonist or PGF_{2α} analogue is at least one selected from the group consisting of latanoprost, bimatoprost, unoprostone, carboprost, tafluprost and travoprost.

3. The pharmaceutical composition of claim 1, wherein the kidney disease is caused by administration of an antibiotic.

4. The pharmaceutical composition of claim 3, wherein the antibiotic is at least one selected from the group consisting of colistin, erythromycin, ampicillin, polymyxin B, tetracycline, kanamycin, neomycin, tylosin, levofloxacin, methicillin, imipenem, amikacin and derivatives thereof.

5. The pharmaceutical composition of claim 1, wherein the kidney disease is at least one selected from the group consisting of renal failure, nephritis, pyelitis, nephrotic syndrome, renal cancer, acute pyelonephritis, chronic pyelonephritis, renal tuberculosis, diabetic nephropathy, chronic glomerulonephritis, rapidly progressive glomerulonephritis, nephrotic syndrome, focal segmental glomerulosclerosis, membranous nephropathy and membranoproliferative glomerulonephritis.

6. The pharmaceutical composition of claim 1, wherein the composition inhibits or prevents generation of reactive oxygen species or apoptosis.

7. The pharmaceutical composition of claim 1, wherein the composition improves or alleviates at least one symptom selected from the group consisting of weight loss, change in kidney color, increased blood urea nitrogen level, increased blood creatinine level, and increased expression of kidney injury marker-1 in a subject.

8. The pharmaceutical composition of claim 1, wherein the composition is administered simultaneously, sequentially, or separately with an antibiotic to a subject.

9. A food composition for alleviating or preventing kidney diseases, comprising, as an active ingredient, at least one selected from the group consisting of PGF_{2α}, a PGF_{2α} agonist and a PGF_{2α} analogue.
